# FASCICULE DE BREVET EUROPEEN

(11) **EP 3 413 813 B1**
(45) Date de publication et mention de la délivrance du brevet: **17.12.2025**
(21) Numéro de dépôt: 17719488.3
(22) Date de dépôt: 09.02.2017
(51) Int. Cl.: A61B 17/221

(54) **DISPOSITIF DE THROMBECTOMIE**
THROMBEKTOMIEVORRICHTUNG
THROMBECTOMY DEVICE

(30) Priorité: 09.02.2016 FR 1650998
(43) Date de publication de la demande: 19.12.2018
(73) Titulaire: Machi, Antonino, 1050 Ixelles (BE)
(72) Inventeur: Machi, Antonino, 1050 Ixelles (BE)
(74) Mandataire: Flesselles, Bruno F.G.
(86) Numéro de dépôt international: PCT/EP2017/052912
(87) Numéro de publication internationale: WO 2017/137508

(56) Documents cités:
- WO-A1-2018/080590
- US-A1- 2012 035 649
- US-A1- 2014 005 717
- US-A1- 2015 025 555
- US-A1- 2015 223 829
- US-A1- 2016 000 450

## Description

### DOMAINE TECHNIQUE DE L'INVENTION

La présente invention vise un dispositif de thrombectomie. Elle s'applique, notamment, aux procédures de thrombectomie endovasculaire.

### ETAT DE LA TECHNIQUE

Une thrombectomie endovasculaire consiste en le retrait mécanique d'un thrombus causant de l'occlusion d'un vaisseau sanguin d'un patient, par exemple dans le cadre de l'accident ischémiques cérébral aigue (AVC). Ce type d'intervention est actuellement réalisé avec de dispositifs de thrombectomie qui présentent par exemple un structure cylindrique type « stent » et un poussoir qui est rattaché à son extrémité proximale, c'est-à-dire l'extrémité la plus proche de l'opérateur. De tels systèmes sont par exemple décrits dans la demande de brevet américaine US 2014/0343595.

Dans les systèmes actuels :
- un fil guide est inséré dans le vaisseau sanguin du patient victime d'une occlusion vasculaire et positionné en aval du thrombus,
- un micro-cathéter est ensuite avancé sur le fil guide au-delà du thrombus,
- le fil guide est retiré et le dispositif de thrombectomie est déplacé conjointement à son poussoir dans le vaisseau sanguin au travers du micro-cathéter et deployé au niveau du thrombus ou juste en aval,
- le dispositif de thrombectomie, toujours attaché au poussoir par son extrémité proximale, est retiré de manière à avoir une interaction avec le thrombus, qui occupe la lumière du vaisseau.

L'interaction entre le dispositif de thrombectomie et le thrombus pendant le retrait permet au dispositif de pénétrer et capturer le thrombus.

Cependant le retrait du dispositif par son extrémité proximale, spécialement en présence de thrombus rigide (Par exemple riche en fibrine ou calcifiées) et/ou vaisseaux tortueux fait que pendant le retrait le dispositif suit la direction de force du poussoir et il s'aplati lors du contact avec le thrombus sans avoir aucun effet de traction sur lui. Dans ces contions le dispositif de thrombectomie ne parvient pas à retirer le thrombus.

D'autre part, certains dispositifs connus, une fois positionnés contre un thrombus et qu'une force de traction leur est appliquée, subissent un renversement que l'on surnomme parfois « une mise en chaussette ». Ce renversement est provoqué par une rigidité trop faible du dispositif qui, confronté à la force de traction, se replie en inversant les faces intérieures et extérieures, de l'autre côté d'une extrémité distale de fixation du dispositif au poussoir.

US 2014/0005717 divulgue des dispositifs de thrombectomie basées sur des structures tressées.

WO 2018/080590, qui fait partie de l'art antérieur suivant l'article 54(3) CBE, divulgue également des dispositifs de thrombectomie basées sur des structures tressées.

### OBJET DE L'INVENTION

La présente invention vise à remédier à tout ou partie de ces inconvénients.

A cet effet, la présente invention, tel que définie par la revendication 1, vise un dispositif de thrombectomie, qui comporte :
- un fil, dit « poussoir », fixé à une structure tressée au niveau de l'extrémité distale de la structure tressée, la structure tressée entourant, au niveau de l'extrémité proximale de ladite structure tressée, une ouverture (115) de diamètre variable en fonction d'une configuration de la structure tressée et
- la structure tressée présentant deux configurations :
   - une configuration déployée, dans laquelle la structure tressée est éloignée radialement du poussoir pour ouvrir l'ouverture entourée par la structure tressée et comprimée axialement en direction de l'extrémité distale et
   - une configuration repliée, dans laquelle la structure tressée est radialement proche du poussoir et étendue axialement le long du poussoir.

Grâce à ces dispositions, pendant le retrait du dispositif, par l'exercice d'une force de traction sur le poussoir, la structure tressée épouse la forme des parois indépendamment du mouvement du poussoir et se comprime axialement, ce qui lui confère une rigidité accrue et la capacité de récolter le thrombus. Ceci permet d'optimiser la capture du thrombus en évitant un aplatissement de la structure tressée. Ainsi, le dispositif ne suit pas la direction de traction du poussoir mais la direction de la paroi du vaisseau sanguin jusqu'à entrer en contact avec le thrombus, ce thrombus étant arraché à la paroi et positionné à l'intérieur de la structure tressée, ce qui en permet l'extraction. La fixation de la structure tressée à une extrémité tressée déployée et comprimé sur l'axe axiale et le cathéter. Ceci éviter que des fragments de thrombus soit libérés dans le flux sanguin lors du retrait du cathéter d'aspiration et du thrombus.

En accord avec l'invention, la structure tressée comporte :
- une première partie présentant un premier diamètre fixé au poussoir et
- une deuxième partie, fixée à la première partie, présentant un deuxième diamètre supérieur au premier diamètre.

Ces modes de réalisation permettent d'améliorer la maîtrise de la forme du déploiement du dispositif lors de la réalisation d'une traction sur le poussoir fixé au dispositif.

En accord avec l'invention, la structure tressée comporte, entre la première et la deuxième partie, une troisième partie de troisième diamètre compris entre le premier et le deuxième diamètre, ce troisième diamètre étant croissant depuis la première vers la deuxième partie.

Cette troisième partie présente un angle de tressage qui permet son expansion par compression axiale pendant le retrait. De plus, l'angle de tressage permet, préférentiellement, au dispositif de ne pas se reverser pendant le retrait. Suivant l'invention, cet angle de tressage est compris entre 65 et 75 degrés.

Ces modes de réalisation permettent d'améliorer la maîtrise de la forme du déploiement du dispositif lors de la réalisation d'une traction sur le poussoir fixé au dispositif.

Dans des modes de réalisation, la première et la troisième partie de la structure tressée présentent un angle de tressage compris entre 65 et 75 degrés.

La force radiale du dispositif est liée à l'angle de tressage : plus important est l'angle de tressage et plus est importante est la force radiale.

Dans des modes de réalisation, la première partie présente une longueur de dix millimètres et la troisième partie de la présente une longueur de cinq millimètres.

Dans des modes de réalisation, la structure tressée comporte une quatrième partie fixée à une extrémité de la deuxième partie, cette quatrième partie présentant un quatrième diamètre inférieur au deuxième diamètre, ce quatrième diamètre étant décroissant depuis la deuxième partie.

Cette quatrième partie, repliée vers la lumière du dispositif, permet au dispositif d'avoir une interaction non traumatique pendant le retrait avec les angles artériels ; qui se trouvent par exemple au niveau des bifurcations artérielles,

Dans des modes de réalisation, la première partie présente une longueur de dix millimètres et la troisième partie de la présente une longueur de cinq millimètres.

Dans des modes de réalisation, la structure tressée comporte une quatrième partie fixée à une extrémité de la deuxième partie, cette quatrième partie présentant un quatrième diamètre inférieur au deuxième diamètre, ce quatrième diamètre étant décroissant depuis la deuxième partie.

Cette quatrième partie, repliée vers la lumière du dispositif, permet au dispositif d'avoir une interaction non traumatique pendant le retrait avec les angles artériels ; qui se trouvent par exemple au niveau des bifurcations artérielles.

Ces modes de réalisation permettent d'améliorer l'expansion du dispositif pendant la traction.

Dans des modes de réalisation, la quatrième partie présente un angle de tressage compris entre 45 et 55 degrés.

La quatrième partie doit avoir une force radiale inférieure par rapport aux autres parties car elle doit pouvoir se reverser un peu notamment pour mieux capturer le thrombus et en même temps pour être non traumatique pour les parois des vaisseaux, ce comportement est dû notamment à l'angle de tressage.

Dans des modes de réalisation, la quatrième partie présente un diamètre, en configuration déployée, de 1,5 millimètres.

Dans des modes de réalisation, la deuxième partie de la structure tressée présentent un angle de tressage compris entre 65 et 75 degrés.

Dans des modes de réalisation, la deuxième partie présente une longueur de six millimètres.

Dans des modes de réalisation, la deuxième partie présente, sur quatre millimètres, à partir de la fixation à la première partie, un angle de tressage compris entre 65 et 75 degrés.

Dans des modes de réalisation, la deuxième partie présente, sur deux millimètres, à partir des quatre millimètres depuis la fixation à la première partie, un angle de tressage compris entre 45 et 55 degrés.

L'angle de tressage des différents segments combiné avec leur longueur permet au dispositif de ne pas s'écraser et de ne pas se reverser face au thrombus pendant le retrait.

Dans des modes de réalisation, la deuxième partie présente un diamètre, en configuration déployée, de deux millimètres.

Les dimensionnements de longueurs et d'angles de tressage de ces modes de réalisation particuliers participent tous à la réduction de l'effet de renversement du dispositif lorsqu'une traction est exercée sur le poussoir.

Dans des modes de réalisation, la structure tressée est réalisée en matériau élastique et à mémoire de forme.

Ainsi, une fois le dispositif positionné à l'intérieur du vaisseau sanguin, la structure tressée prend la configuration déployée spontanément.

Dans des modes de réalisation, au moins une partie de la structure tressée est recouverte par un revêtement de platine.

Ces modes de réalisation ont l'avantage de rendre radio-opaque le dispositif.

Dans des modes de réalisation, le poussoir comporte un point de fixation distal à la structure tressée, cette structure tressée comportant des fils coulissants les uns sur les autres de manière à former des mailles de dimensions variables selon l'état d'ouverture de la structure tressée.

Ces modes de réalisation permettent aux fils de coulisser pour passer d'une configuration à l'autre.

Dans des modes de réalisation particuliers, la structure tressée est formée à partir d'entre 4 et 400 fils tressés.

Dans des modes de réalisation particuliers, la structure tressée est formée à partir d'entre 10 et 100 fils tressés.

Dans des modes de réalisation particuliers, la structure tressée est formée à partir d'entre 20 et 50 fils tressés.

Préférentiellement, 36 fils sont utilisés pour former ladite structure.

Dans des modes de réalisation particuliers, chaque fil de la structure présente un diamètre compris entre 1 et 100 micromètres.

Dans des modes de réalisation particuliers, chaque fil de la structure présente un diamètre compris entre 10 et 80 micromètres.

Dans des modes de réalisation particuliers, chaque fil de la structure présente un diamètre compris entre 15 et 50 micromètres.

Préférentiellement, le diamètre des fils est égal à 30 micromètres.

### BREVE DESCRIPTION DES FIGURES

D'autres avantages, buts et caractéristiques particulières de l'invention ressortiront de la description non limitative qui suit d'au moins un mode de réalisation particulier du dispositif et du procédé objets de la présente invention, en regard des dessins annexés, dans lesquels :
- la figure 1 représente, schématiquement et en coupe, un mode de réalisation particulier du dispositif objet de la présente invention,
- la figure 2 représente, schématiquement et en coupe, un mode de réalisation particulier du dispositif objet de la présente invention,
- la figure 3 représente, schématiquement et en perspective, un mode de réalisation particulier du dispositif objet de la présente invention,
- les figures 4 à 7 représentent, schématiquement et en coupe, une application particulière du mode de réalisation particulier du dispositif, et
- la figure 8 représente, schématiquement et sous forme d'un logigramme, une succession d'étapes particulière du procédé qui ne fait pas partie de la présente invention.

### DESCRIPTION D'EXEMPLES DE REALISATION DE L'INVENTION

La présente description est donnée à titre non limitatif, chaque caractéristique d'un mode de réalisation pouvant être combinée à toute autre caractéristique de tout autre mode de réalisation de manière avantageuse.

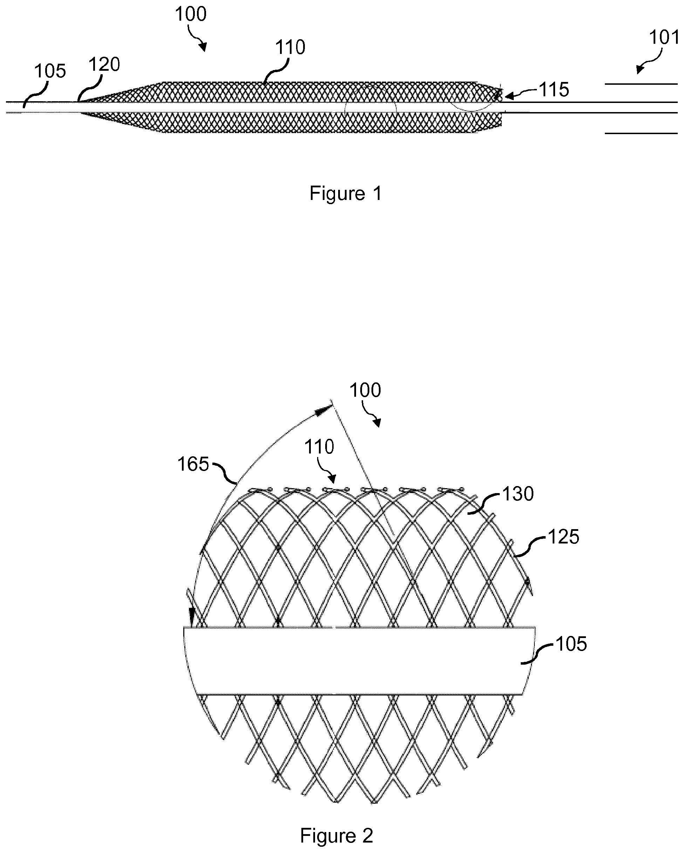

On observe, sur les figures 1 à 3, qui ne sont pas à l'échelle, une vue schématique d'un mode de réalisation du dispositif 100 objet de la présente invention. Ce dispositif 100 de thrombectomie comporte :
- un fil 105, dit « poussoir », fixé à une structure tressée 110 au niveau de l'extrémité distale de la structure tressée, la structure 110 tressée entourant, au niveau de l'extrémité proximale de ladite structure 110 tressée, une ouverture 115 de diamètre variable en fonction d'une configuration de la structure 110 tressée et
- la structure 110 tressée présentant deux configurations :
   - une configuration déployée, dans laquelle la structure tressée est éloignée radialement du poussoir pour ouvrir l'ouverture entourée par la structure tressée et, pendant qu'une traction est réalisée sur le poussoir, comprimée axialement en direction de l'extrémité distale et
   - une configuration repliée, dans laquelle la structure tressée est radialement proche du poussoir et étendue axialement le long du poussoir, en absence de traction sur ledit poussoir.

Le poussoir 105 est, par exemple, un fil en acier inoxydable présentant un diamètre d'environ 0,35 millimètres et une longueur d'environ deux mètres. Préférentiellement, ce fil est rendu radio-opaque par l'adjonction d'un revêtement de platine.

Ce poussoir 105 est fixé, à proximité de l'extrémité distale de la structure tressée 110.

Cette structure tressée 110 est formé de fils tressés présentant un diamètre de 30 ou 32 micromètres par exemple. Cette structure est formée de 4 à 400 fils, et préférentiellement 36. Cette structure tressée 110 permet aux fils de coulisser les uns sur les autres, ce qui permet une expansion radiale et une compression axiale optimale du structure tressée 110 pendant le retrait du dispositif 100 du vaisseau sanguin.

Les fils formant la structure 110 tressée présentent une certaine élasticité et sont formés préférentiellement d'un matériau élastique et à mémoire de forme tel, par exemple, d'un alliage de titane et de nickel, dit « Nitinol », ou de chrome et de cobalt. Ainsi, dès le positionnement du dispositif 100 dans un vaisseau sanguin, la structure 110 tressée s'étend pour entrer au contact des parois du vaisseau sanguin.

Cette structure tressée 110 présente deux extrémités :
- l'une est dite « distale », cette extrémité étant fixée à proximité de l'extrémité distale du poussoir 105 et
- l'autre est dite « proximale », cette extrémité étant plus éloignée de l'extrémité distale du poussoir 105 que l'extrémité distale de la structure tressée 110.

De cette manière, l'extrémité distale de la structure tressée 110 est positionnée en aval de l'extrémité proximale de la structure tressée 110 par rapport au thrombus lorsque la structure tressée 110 est intégralement situé en aval du thrombus par rapport au flux sanguin traversant le vaisseau sanguin.

La structure tressée 110 entoure, au niveau de l'extrémité proximale de la structure tressée 110, une ouverture 115 destinée à permettre le passage du thrombus lorsque la structure tressée 110 est en configuration déployée pendant le retrait du dispositif 100.

Ainsi, comme on le comprend, l'exercice d'une force de traction sur le poussoir, c'est-à-dire d'une force visant à déplacer le poussoir 105 vers une lumière réalisée dans le vaisseau sanguin, provoque le déploiement du structure tressée 110 et une compression axiale.

Au cours de ce retrait, la structure tressée 110 subit une expansion radiale et une compression axiale en conséquence.

Cette structure tressée 110 présente, par exemple, une longueur de vingt - cinq millimètres en configuration repliée, c'est-à-dire au repos. Cette structure tressée 110 présente, par exemple, un diamètre de deux millimètres en configuration déployée.

On appelle « angle de tressage » l'angle formé entre :
- la projection de la tangente d'un point d'un fil de la structure tressée 110 sur un plan perpendiculaire au rayon de la structure tressée 110 passant par ce point et par l'axe central de symétrie de la structure tressée 110 et
- l'axe central de la structure tressée 110.

La fixation de la structure tressée 110 au poussoir 105 est réalisée, par exemple, par soudage, collage ou sertissage ou avec un outil mécanique.

Préférentiellement, le poussoir 105 comporte un point 120 de fixation distale à la structure tressée 110, cette structure tressée comportant des fils 125 coulissants les uns sur les autres de manière à former des mailles 130 de dimensions variables selon l'état d'ouverture de la structure tressée.

Les dimensions de ces mailles sont réduites lorsque le dispositif 100 est en configuration déployée.

Préférentiellement, le poussoir 105 et la structure tressée 110 sont introduits dans le vaisseau sanguin par l'utilisation d'un micro-cathéter 102. Ce micro-cathéter 102 présente, par exemple, un diamètre inférieur à deux millimètres.

Préférentiellement, la structure tressée 110 comporte :
- une première partie 135 présentant un premier diamètre fixé au poussoir 105 et
- une deuxième partie 140, fixée à la première partie 135, présentant un deuxième diamètre supérieur au premier diamètre.

Le premier diamètre est, par exemple, constant le long du dispositif 100 et fixé sur toute la longueur au poussoir 105 de manière à en renforcer la fixation.

Ce premier diamètre est, par exemple, égal à 0,35 millimètres.

La première partie 135 présente par exemple une longueur d'un centimètre.

La deuxième partie 140 présente un deuxième diamètre variable en fonction de la configuration de la structure tressée 110. En tout état de cause, ce deuxième diamètre est supérieur ou égal au premier diamètre quelle que soit la configuration dans laquelle est la structure tressée 110.

La deuxième partie 140 présente, par exemple, une longueur de 6 millimètres.

Préférentiellement, la structure tressée 110 comporte entre la première et la deuxième partie, 135 et 140, une troisième partie 145 de troisième diamètre compris entre le premier et le deuxième diamètre, ce troisième diamètre étant croissant depuis la première 135 vers la deuxième partie 140.

La troisième partie 145 présente, par exemple, une longueur de cinq millimètres. Cette troisième partie 145 présente, au niveau du contact avec la première partie 135, un diamètre égal au diamètre de la première partie 135 et, au niveau du contact avec la deuxième partie 140, un diamètre égal au diamètre de la deuxième partie 140.

Préférentiellement, la première et la troisième partie, 135 et 145, de la structure tressée présentent un angle de tressage compris entre 65 et 75 degrés. Préférentiellement, cet angle est égal à 70 degrés.

La première partie 135 présente une longueur de dix millimètres et la troisième partie 145 de la présente une longueur de cinq millimètres.

Préférentiellement, la deuxième partie 140 de la structure tressée présente un angle de tressage compris entre 65 et 75 degrés. Préférentiellement, cet angle est égal à 70 degrés.

Préférentiellement, la deuxième partie 140 présente une longueur de six millimètres.

Préférentiellement, la deuxième partie 140 présente, sur quatre millimètres, à partir de la fixation à la première partie 135, un angle de tressage compris entre 65 et 75 degrés. Préférentiellement, cet angle est égal à 70 degrés.

Préférentiellement, la deuxième partie 140 présente, sur deux millimètres, à partir des quatre millimètres depuis la fixation à la première partie 135, un angle de tressage compris entre 45 et 55 degrés. Préférentiellement, cet angle est égal à 50 degrés.

Préférentiellement, la deuxième partie 140 présente un diamètre, en configuration déployée, de deux millimètres.

Ces dimensionnements permettent au dispositif de présenter une force radiale et structurelle empêchant tout renversement.

Préférentiellement, la structure tressée 110 comporte une quatrième partie 150 fixée à une extrémité de la deuxième partie 140, cette quatrième partie 150 présentant un quatrième diamètre inférieur au deuxième diamètre, ce quatrième diamètre étant décroissant depuis la deuxième partie 140.

La quatrième partie 150 présente, par exemple, une longueur de quatre millimètres.

Préférentiellement, la quatrième partie 150 présente un angle de tressage compris entre 45 et 55 degrés. Préférentiellement, cet angle de tressage est égal à 50 degrés.

Préférentiellement, la quatrième partie 150 présente un diamètre, en configuration déployée, de 1,5 millimètres.

Ces dimensionnements permettent au dispositif de présenter à l'extrémité formée par la quatrième partie, une rigidité moindre permettant de limiter les risques d'accrochage avec les parois du vaisseau sanguin.

Dans des modes de réalisation, le dispositif 100 est associé à un cathéter 101 d'aspiration présentant une valeur de diamètre déterminée dans lequel le poussoir est inséré, la structure tressée, présentant, lors de la configuration déployée, un diamètre grossièrement égal à ladite valeur de diamètre.

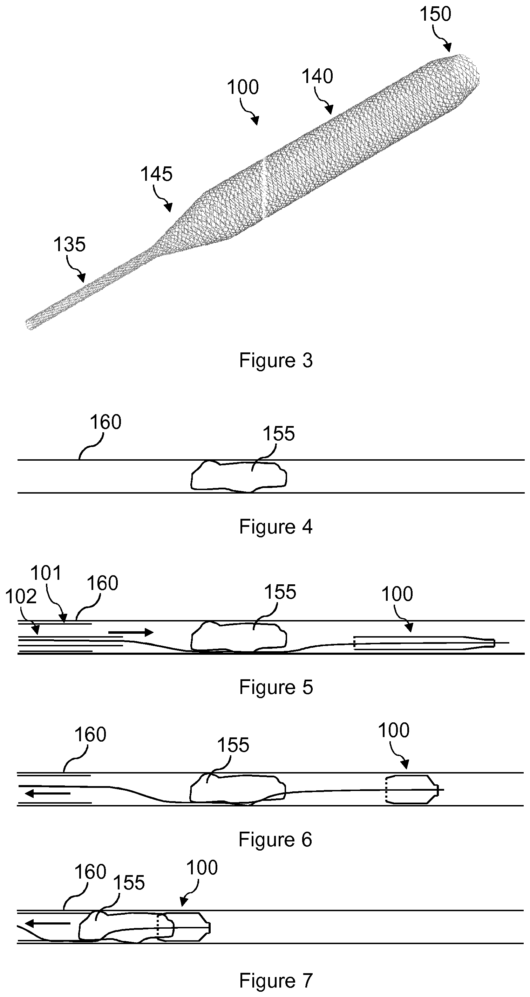

On observe, en figures 4 à 7, successivement, différentes étapes du retrait d'un thrombus par le dispositif 100.

En particulier, on observe :
- en figure 4, un vaisseau 160 sanguin obstrué par un thrombus 155,
- en figure 5, le dispositif 100 inséré dans le vaisseau 160 sanguin en amont du thrombus 155, par rapport au flux de circulation sanguine, et dépassant le thrombus 155 de manière à être positionné en aval dudit thrombus 155,
- en figure 6, la structure tressée déployée et comprimé sur le plan axial par un mouvement de traction exercé sur le fil et le dispositif 100 suivant une direction de mouvement diffèrent par rapport au poussoir 105,
- en figure 7, le thrombus 155 capturé par la structure 115 tressée, ce thrombus pouvant être librement extrait du vaisseau 160 sanguin par retrait du dispositif 100 ou approché au cathéter d'aspiration.

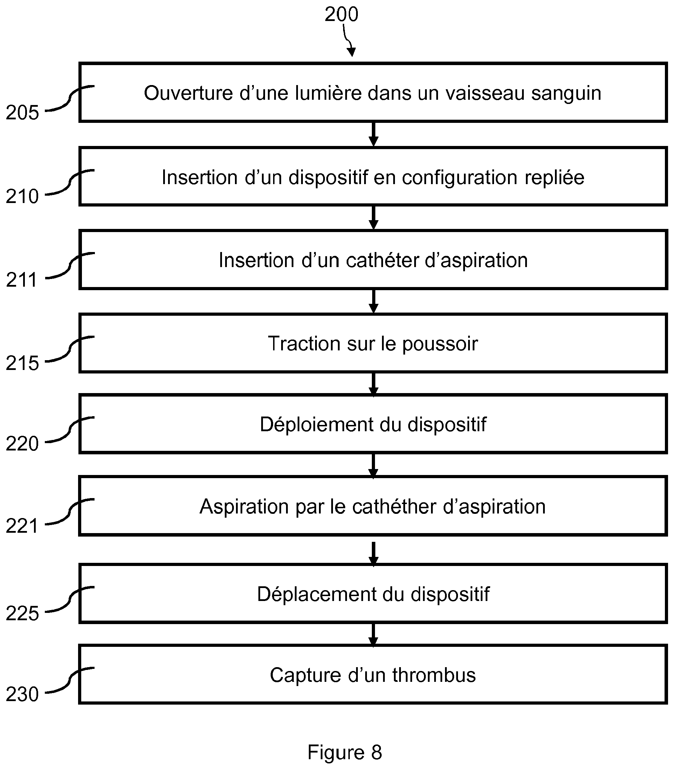

On observe, en figure 8, schématiquement, un mode de réalisation particulier du procédé 200 correspondant aux étapes illustrées en figures 4 à 7. Ce procédé 200 de thrombectomie comporte :
- une étape 205 d'ouverture d'une lumière dans un vaisseau sanguin en amont du thrombus à opérer par rapport au flux sanguin circulant dans le vaisseau sanguin,
- une étape 210 d'insertion d'un dispositif 100, tel que décrit en regard des figures 1 à 3, dans le vaisseau sanguin, au moyen d'un micro-cathéter 102 par exemple, la structure tressée de ce dispositif 100 étant en configuration repliée,
- une étape d'extraction du dispositif 100 du micro-cathéter 102 pour que le dispositif soit positionné en aval du thrombus et pour que la structure 110 tressée se déploie par mémoire de forme,
- optionnellement, une étape 211 d'insertion d'un cathéter d'aspiration 101 entourant le micro-cathéter, cette étape pouvant être réalisée en amont ou en aval de l'étape d'insertion 210,
- une étape 215 de traction sur le fil pour provoquer un déploiement optimal 220 et le déplacement 225 simultané du dispositif 100 le long des parois du vaisseau sanguin, quelle que soit la traction réalisée sur le poussoir,
- optionnellement, une étape 221 d'aspiration réalisée par le cathéter d'aspiration 101, cette étape d'aspiration 221 pouvant être réalisée en amont ou en aval de l'étape de traction 215 et
- une étape 230 de capture du dispositif dans l'ouverture entourée par la structure tressée.

## Revendications

1. Dispositif (100) de thrombectomie, **caractérisé en ce qu'**il comporte :
- un fil (105), dit « poussoir », fixé à une structure tressée (110) au niveau de l'extrémité distale de la structure tressée, la structure tressée entourant, au niveau de l'extrémité proximale de ladite structure tressée, une ouverture (115) de diamètre variable en fonction d'une configuration de la structure tressée et
- la structure tressée présentant deux configurations :
- une configuration déployée, dans laquelle la structure tressée est éloignée radialement du poussoir pour ouvrir l'ouverture entourée par la structure tressée et comprimée axialement en direction de l'extrémité distale et
- une configuration repliée, dans laquelle la structure tressée est radialement proche du poussoir et étendue axialement le long du poussoir,
la structure tressée (110) comportant :
- une première partie (135) fixée au poussoir (105) et présentant un premier diamètre et
- une deuxième partie (140), reliée à la première partie, présentant un deuxième diamètre supérieur au premier diamètre, et
- entre la première et la deuxième partie (135, 140), une troisième partie (145) de troisième diamètre compris entre le premier et le deuxième diamètre, ce troisième diamètre étant croissant depuis la première vers la deuxième partie, la troisième partie présentant un angle de tressage qui permet son expansion par compression axiale pendant le retrait, **caractérisé par** l'angle de tressage étant compris entre 65 et 75 degrés.

2. Dispositif selon la revendication 1, **caractérisé en ce que** la structure tressée comprimée axialement en direction de l'extrémité distale pendant qu'une traction est réalisée sur le poussoir et est radialement proche du poussoir et étendue axialement le long du poussoir, en absence de traction sur ledit poussoir.

3. Dispositif (100) selon la revendication 1 ou 2, qui est associé à un cathéter (101) d'aspiration présentant une valeur de diamètre déterminée dans lequel le poussoir est inséré, la structure tressée, présentant, lors de la configuration déployée, un diamètre grossièrement égal à ladite valeur de diamètre.

4. Dispositif (100) selon l'une des revendications 1 à 3, dans lequel la première partie (135, 145) de la structure tressée présente un angle de tressage compris entre 65 et 75 degrés.

5. Dispositif (100) selon l'une des revendications 1 à 4, dans lequel la première partie (135) présente une longueur de dix millimètres et la troisième partie (145) de la présente une longueur de cinq millimètres.

6. Dispositif (100) selon l'une des revendications 1 à 5, dans lequel la structure tressée comporte une quatrième partie (150) fixée à une extrémité de la deuxième partie (140), cette quatrième partie présentant un quatrième diamètre inférieur au deuxième diamètre, ce quatrième diamètre étant décroissant depuis la deuxième partie.

7. Dispositif (100) selon la revendication 6, dans lequel la quatrième partie (150) présente un angle de tressage compris entre 45 et 55 degrés.

8. Dispositif (100) selon l'une des revendications 6 à 7, dans lequel la quatrième partie (150) présente un diamètre, en configuration déployée, de 1,5 millimètres.

9. Dispositif (100) selon l'une des revendications 1 à 8, dans lequel la deuxième partie (140) de la structure tressée présente un angle de tressage compris entre 65 et 75 degrés.

10. Dispositif (100) selon l'une des revendications 1 à 9, dans lequel la deuxième partie (140) présente une longueur de six millimètres.

11. Dispositif (100) selon l'une des revendications 9 ou 10, dans lequel la deuxième partie (140) présente, sur quatre millimètres, à partir de la fixation à la première partie (135), un angle de tressage compris entre 65 et 75 degrés.

12. Dispositif (100) selon la revendication 11, dans lequel la deuxième partie (140) présente, sur deux millimètres, à partir des quatre millimètres depuis la fixation à la première partie (135), un angle de tressage compris entre 45 et 55 degrés.

13. Dispositif (100) selon l'une des revendications 1 à 12, dans lequel la deuxième partie (140) présente un diamètre, en configuration déployée, de deux millimètres.

14. Dispositif (100) selon l'une des revendications 1 à 13, dans lequel la structure tressée est réalisée en matériau élastique et à mémoire de forme.

15. Dispositif (100) selon l'une des revendications 1 à 14, dans lequel au moins une partie de la structure tressée (110) est recouverte par un revêtement de platine.

16. Dispositif (100) selon l'une des revendications 1 à 15, dans lequel le poussoir comporte un point (120) de fixation distale à la structure tressée (110), cette structure tressée comportant des fils (125) coulissants les uns sur les autres de manière à former des mailles (130) de dimensions variables selon l'état d'ouverture de la structure tressée.

17. Dispositif (100) selon l'une des revendications 1 à 16, dans lequel la structure tressée est formée à partir d'entre 4 et 400 fils tressés.

18. Dispositif (100) selon l'une des revendications 1 à 17, dans lequel chaque fil de la structure présente un diamètre compris entre 1 et 100 micromètres.

## Patentansprüche

1. Thrombektomievorrichtung (100), **dadurch gekennzeichnet, dass** sie umfasst:
- einen als "Schieber" bezeichneten Draht (105), der an einer geflochtenen Struktur (110) am distalen Ende der geflochtenen Struktur befestigt ist, wobei die geflochtene Struktur am proximalen Ende der geflochtenen Struktur eine Öffnung (115) mit einem Durchmesser umgibt, der je nach Konfiguration der geflochtenen Struktur variiert, und
- die geflochtene Struktur zwei Konfigurationen aufweist:
- eine ausgefahrene Konfiguration, in der die geflochtene Struktur radial vom Drücker entfernt ist, um die von der geflochtenen Struktur umgebene Öffnung zu öffnen, und axial in Richtung des distalen Endes zusammengedrückt ist, und
- eine zusammengeklappte Konfiguration, in der die geflochtene Struktur radial nahe am Drücker liegt und axial entlang des Drückers ausgestreckt ist,
wobei die geflochtene Struktur (110) umfasst:
- einen ersten Abschnitt (135), der am Drücker (105) befestigt ist und einen ersten Durchmesser aufweist, und
- einen zweiten Abschnitt (140), der mit dem ersten Abschnitt verbunden ist und einen zweiten Durchmesser aufweist, der größer ist als der erste Durchmesser, und
- zwischen dem ersten und dem zweiten Abschnitt (135, 140) einen dritten Abschnitt (145) mit einem dritten Durchmesser, der zwischen dem ersten und dem zweiten Durchmesser liegt, wobei dieser dritte Durchmesser vom ersten zum zweiten Abschnitt zunimmt, wobei der dritte Abschnitt einen Flechtwinkel aufweist, der seine Ausdehnung durch axiale Kompression während des Zurückziehens ermöglicht, **dadurch gekennzeichnet, dass** der Flechtwinkel zwischen 65 und 75 Grad liegt.

2. Vorrichtung nach Anspruch 1, **dadurch gekennzeichnet, dass** die geflochtene Struktur in axialer Richtung zum distalen Ende hin zusammengedrückt wird, während eine Zugkraft auf den Schieber ausgeübt wird, und sich in radialer Richtung nahe am Schieber und in axialer Richtung entlang des Schiebers erstreckt, wenn keine Zugkraft auf den Schieber ausgeübt wird.

3. Vorrichtung (100) nach Anspruch 1 oder 2, die mit einem Saugkatheter (101) mit einem bestimmten Durchmesser verbunden ist, in den der Schieber eingeführt wird, wobei die geflochtene Struktur im entfalteten Zustand einen Durchmesser aufweist, der in etwa dem Durchmesser entspricht.

4. Vorrichtung (100) nach einem der Ansprüche 1 bis 3, wobei der erste Teil (135, 145) der geflochtenen Struktur einen Flechtwinkel zwischen 65 und 75 Grad aufweist.

5. Vorrichtung (100) nach einem der Ansprüche 1 bis 4, wobei der erste Teil (135) eine Länge von zehn Millimetern und der dritte Teil (145) eine Länge von fünf Millimetern aufweist.

6. Vorrichtung (100) nach einem der Ansprüche 1 bis 5, wobei die geflochtene Struktur einen vierten Teil (150) aufweist, der an einem Ende des zweiten Teils (140) befestigt ist, wobei dieser vierte Teil einen vierten Durchmesser aufweist, der kleiner ist als der zweite Durchmesser, wobei dieser vierte Durchmesser vom zweiten Teil aus abnimmt.

7. Vorrichtung (100) nach Anspruch 6, wobei der vierte Teil (150) einen Flechtwinkel zwischen 45 und 55 Grad aufweist.

8. Vorrichtung (100) nach einem der Ansprüche 6 bis 7, wobei der vierte Teil (150) in ausgebreiteter Konfiguration einen Durchmesser von 1,5 Millimetern aufweist.

9. Vorrichtung (100) nach einem der Ansprüche 1 bis 8, wobei der zweite Teil (140) der geflochtenen Struktur einen Flechtwinkel zwischen 65 und 75 Grad aufweist.

10. Vorrichtung (100) nach einem der Ansprüche 1 bis 9, wobei der zweite Teil (140) eine Länge von sechs Millimetern aufweist.

11. Vorrichtung (100) nach einem der Ansprüche 9 oder 10, wobei der zweite Teil (140) über vier Millimeter, gemessen von der Befestigung am ersten Teil (135), einen Flechtwinkel zwischen 65 und 75 Grad aufweist.

12. Vorrichtung (100) nach Anspruch 11, wobei der zweite Teil (140) über zwei Millimeter, ausgehend von den vier Millimetern ab der Befestigung am ersten Teil (135), einen Flechtwinkel zwischen 45 und 55 Grad aufweist.

13. Vorrichtung (100) nach einem der Ansprüche 1 bis 12, wobei der zweite Teil (140) in ausgebreiteter Konfiguration einen Durchmesser von zwei Millimetern aufweist.

14. Vorrichtung (100) nach einem der Ansprüche 1 bis 13, wobei die geflochtene Struktur aus elastischem Material mit Formgedächtnis hergestellt ist.

15. Vorrichtung (100) nach einem der Ansprüche 1 bis 14, wobei mindestens ein Teil der geflochtenen Struktur (110) mit einer Platinbeschichtung überzogen ist.

16. Vorrichtung (100) nach einem der Ansprüche 1 bis 15, wobei der Drücker einen distalen Befestigungspunkt (120) an der geflochtenen Struktur (110) aufweist, wobei diese geflochtene Struktur Drähte (125) aufweist, die aufeinander gleiten, um Maschen (130) mit variablen Abmessungen entsprechend dem Öffnungszustand der geflochtenen Struktur zu bilden.

17. Vorrichtung (100) gemäß einem der Ansprüche 1 bis 16, wobei die geflochtene Struktur aus zwischen 4 und 400 geflochtenen Fäden gebildet ist.

18. Vorrichtung (100) gemäß einem der Ansprüche 1 bis 17, wobei jeder Faden der Struktur einen Durchmesser zwischen 1 und 100 Mikrometern aufweist.

## Claims

1. A thrombectomy device (100), **characterized in that** it has:
- a wire (105), called "pusher" attached to a braided structure (110) at the distal end of the braided structure, the braided structure surrounding, at the proximal end of said braided structure, an opening (115) of variable diameter depending on a configuration of the braided structure and
- the braided structure having two configurations:
- a deployed configuration, in which the braided structure is radially distant from the pusher to open the opening surrounded by the braided structure and compressed axially in the direction of the distal end and
- a folded configuration, in which the braided structure is radially close to the pusher and extends axially along the pusher,
the braided structure (110) comprising:
- a first part (135) attached to the pusher (105) having a first diameter and
- a second part (140), attached to the first part, having a second diameter greater than the first diameter, and
- between the first and the second part (135, 140), a third part (145) of a third diameter comprised between the first and second diameter, this third diameter increasing from the first to the second part, the third part having a braiding angle that allows it to expand by axial compression during withdrawal, **characterized by** the braiding angle being between 65 and 75 degrees.

2. Device according to claim 1, **characterized in that** the braided structure is compressed axially toward the distal end while traction is applied to the pusher and is radially close to the pusher and extended axially along the pusher in the absence of traction on said pusher.

3. The device (100) as claimed in claim 1 or 2, which is associated with a suction catheter (101) with a specified diameter value into which the pusher is inserted, the braided structure having, in the deployed configuration, a diameter roughly equal to said diameter value.

4. The device (100) as claimed in any one of claims 1 to 3, in which the first part (135, 145) of the braided structure has a braiding angle comprised between 65 and 75 degrees.

5. The device (100) as claimed in one of claims 1 to 4, in which the first part (135) has a length of ten millimeters and the third part (145) has a length of five millimeters.

6. The device (100) as claimed in one of claims 1 to 5, in which the braided structure has a fourth part (150) attached to one end of the second part (140), this fourth part having a fourth diameter less than the second diameter, this fourth diameter decreasing from the second part.

7. The device (100) as claimed in claim 6, in which the fourth part (150) has a braiding angle comprised between 45 and 55 degrees.

8. The device (100) as claimed in one of claims 6 to 7, in which the fourth part (150) has a diameter, in deployed configuration, of 1.5 millimeters.

9. The device (100) as claimed in one of claims 1 to 8, in which the second part (140) of the braided structure has a braiding angle comprised between 65 and 75 degrees.

10. The device (100) as claimed in one of claims 1 to 9, in which the second part (140) has a length of six millimeters.

11. The device (100) as claimed in one of claims 9 or 10, in which the second part (140) has, over four millimeters, from the attachment to the first part (135), a braiding angle comprised between 65 and 75 degrees.

12. The device (100) as claimed in claim 11, in which the second part (140) has, over two millimeters, from four millimeters from the attachment to the first part (135), a braiding angle comprised between 45 and 55 degrees.

13. The device (100) as claimed in one of claims 1 to 12, in which the second part (140) has a diameter, in deployed configuration, of two millimeters.

14. The device (100) as claimed in one of claims 1 to 13, in which the braided structure is made of an elastic material with shape memory.

15. The device (100) as claimed in one of claims 1 to 14, in which at least a part of the braided structure (110) is covered by a platinum coating.

16. The device (100) as claimed in one of claims 1 to 15, in which the pusher has a distal attachment point (120) to the braided structure (110), this braided structure having wires (125) sliding over one another so as to form meshes (130) of variable dimensions depending on the opening status of the braided structure.

17. The device (100) as claimed in one of claims 1 to 16, in which the braided structure is formed from between 4 and 400 braided wires.

18. The device (100) as claimed in one of claims 1 to 17, in which each wire of the structure has a diameter comprised between 1 and 100 micrometers.
